# FASCICULE DE BREVET EUROPEEN

(11) **EP 1 420 029 B1**
(45) Date de publication et mention de la délivrance du brevet: **20.02.2008**
(21) Numéro de dépôt: 03293084.4
(22) Date de dépôt: 10.12.2003
(51) Int. Cl.: C07K 5/06

(54) **Procédé de synthèse du perindopril et ses sels pharmaceutiquement acceptables**
Verfahren für die Synthese von Perindopril und seiner pharmazeutisch annehmbaren Salzen
Method for synthesis of perindopril and its pharmaceutically acceptable salts

(43) Date de publication de la demande: 19.05.2004
(73) Titulaire: Les Laboratoires Servier, 92415 Courbevoie Cédex (FR)
(72) Inventeur: Dubuffet, Thierry, 76190 Autretot (FR); Lecouve, Jean-Pierre, 76600 Le Havre (FR)

(56) Documents cités:
- WO-A-01/58868
- LI, PENG ET AL: "New and highly efficient immonium-type peptide coupling reagents: synthesis, mechanism, and application" TETRAHEDRON (2000), 56(26), 4437-4445 , XP004202135
- LI, PENG ET AL: "BOMI - a novel peptide coupling reagent" TETRAHEDRON LETTERS (1999), 40(18), 3605-3608 , XP004162348
- LI P ET AL: "THE DEVELOPMENT OF HIGHLY EFFICIENT ONIUM-TYPE PEPTIDE COUPLING REAGENTS BASED UPON RATIONAL MOLECULAR DESIGN" JOURNAL OF PEPTIDE RESEARCH, MUNKSGAARD INTERNATIONAL PUBLISHERS, COPENHAGEN, DK, vol. 58, no. 2, 1 août 2001 (2001-08-01), pages 129-139, XP001039108 ISSN: 1397-002X
- COSTE, JACQUES ET AL: "Oxybenzotriazole free peptide coupling reagents for N-methylate amino acids" TETRAHEDRON LETTERS (1991), 32(17), 1967-70 , XP001180312
- CHEN, SHAOQING ET AL: "A new coupling reagent for peptide synthesis. Benzotriazolyloxybis(pyrrolidino)carbonium hexafluorophosphate (BBC)" TETRAHEDRON LETTERS (1992), 33(5), 647-50, XP001180313
- CARPINO, LOUIS A. ET AL: "Effect of Tertiary Bases on O-Benzotriazolyluronium Salt-Induced Peptide Segment Coupling" JOURNAL OF ORGANIC CHEMISTRY (1994), 59(4), 695-8, XP002273951

## Description

La présente invention concerne un procédé de synthèse du perindopril de formule (I) : 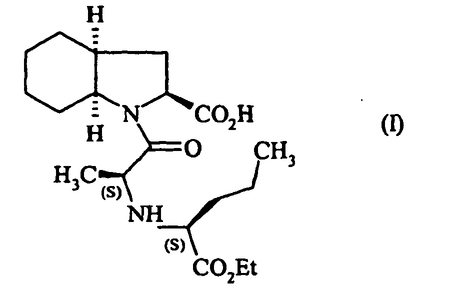 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse performant, facilement transposable à l'échelle industrielle, conduisant au perindopril avec un bon rendement, et surtout avec une excellente pureté.
Le brevet EP 0 308 341 décrit la synthèse industrielle du perindopril par couplage de l'ester benzylique de l'acide (2*S*, 3a*S*, 7a*S*)-octahydroindole 2-carboxylique avec l'ester éthylique de la N-[(*S*)-1-carboxybutyl]-(*S*)-alanine en présence de dicyclohexylcarbodiimide, suivie de la déprotection du groupement carboxylique de l'hétérocycle par hydrogénation catalytique.
Ce procédé présente des inconvénients liés à l'utilisation du dicyclohexylcarbodiimide.

La Demanderesse a mis au point un procédé de synthèse du perindopril qui utilise d'autres agents de couplage.

Plus spécifiquement, la présente invention concerne un procédé de synthèse du perindopril caractérisé en ce que l'on met en réaction l'ester benzylique de formule (IIa) ou (IIb) : 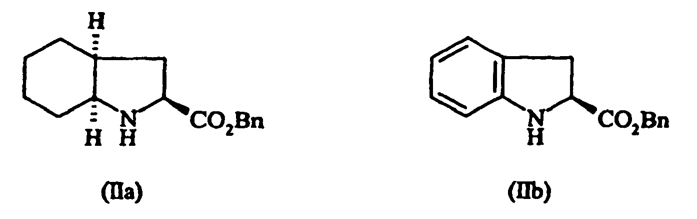 ou le sel d'addition de l'ester de formule (IIa) ou (IIb) avec un acide minéral ou organique,
avec le composé de formule (III): 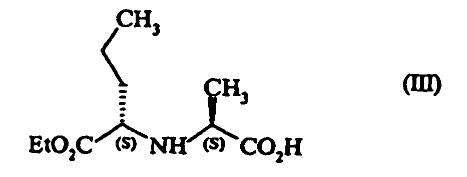 en présence d'un agent de couplage choisi parmi les réactifs et couples de réactifs suivants :
1-(3-diméthylaminopropyl)-3-éthyl-carbodiimide chlorhydrate /1-hydroxybenzotriazole, et anhydride propanephosphonique,
en présence éventuelle d'une base,
pour conduire après hydrogénation catalytique en présence de palladium au perindopril de formule (I), que l'on transforme, si on le souhaite, en un sel pharmaceutiquement acceptable tel que le sel de tert-butylamine.

Lorsqu'on part du composé de formule (IIa), l'hydrogénation catalytique est préférentiellement effectuée sous une pression d'hydrogène inférieure à 10 bars.

Lorsqu'on part du composé de formule (IIb), l'hydrogénation catalytique est préférentiellement effectuée sous une pression d'hydrogène comprise entre 10 et 35 bars.

L'exemple ci-dessous illustre l'invention.

### Référence Exemple 1 : (2S, 3aS, 7aS)-1-{(1S)-1-1(1S)-[-(Ethoxycarbonyl)-butylamino]-propionyl}-octahydro-1H-indole-2-carboxylate de benzyle :

Dans un réacteur sous agitation sont introduits 200 g du paratoluènesulfonate de l'ester benzylique de l'acide (2*S*, 3a*S*, 7a*S*)-octahydroindole 2-carboxylique, 65 ml de triéthylamine, 1 l d'acétate d'éthyle puis, après 10 mn d'agitation à température ambiante, 100 g de N-[(*S*)-carbéthoxy-1-butyl]-(*S*)-alanine, et 175 g de O-(benzotriazol-1-yl)-1,1,3,3-bis(tétraméthylène)-uronium hexafluorophosphate. Le mélange hétérogène est ensuite porté à 30°C pendant 3h sous bonne agitation, puis il est refroidi à 0°C et filtré.
Le filtrat est ensuite lavé, puis évaporé à sec pour conduire au produit attendu.

### Exemple 2 : Acide (2S, 3aS, 7aS)-1-{(2S)-1-[(1S)-1-(éthoxycarbonyl)-butylamino]-propionyl}-octahydro-1H-indok-2-carboxylique :

Le résidu obtenu dans le stade précédent (200 g) est mis en solution dans 200 ml de méthylcyclohexane et transféré dans un hydrogénateur, puis 26 g de charbon palladié à 5% en suspension dans 80 ml de méthylcyclohexane sont ajoutés, suivis de 640 ml d'eau.
Le mélange est ensuite hydrogéné sous une pression de 0,5 bar, à une température comprise entre 15 et 30°C, jusqu'à absorption de la quantité théorique d'hydrogène.
Après filtration du catalyseur, la phase aqueuse du filtrat est lavée par du méthylcyclohexane, puis lyophilisée pour conduire au produit attendu avec un rendement de 94%.

### Exemple 3 : Sel de tert-butylamine de l'acide (2S, 3aS, 7aS)-1-{(2S)-2-[(1S)-1-(étlroxycarbonyl)-butylamino]-propionyl}-octahydro-1H-indole 2-carboxylique :

Le lyophilisat obtenu dans le stade précédent (200 g) est mis en solution dans 2,8 l d'acétate d'éthyle, puis 44 g de tert-butylamine et 400 ml d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95%.

## Revendications

1. Procédé de synthèse industrielle du perindopril de formule (I) 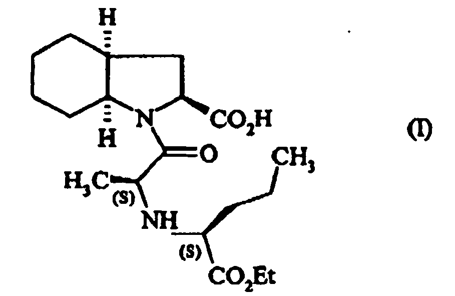 et de ses sels pharmaceutiquement acceptables, **caractérisé en ce que** l'on met en réaction l'ester benzylique de formule (IIa) ou (IIb) : 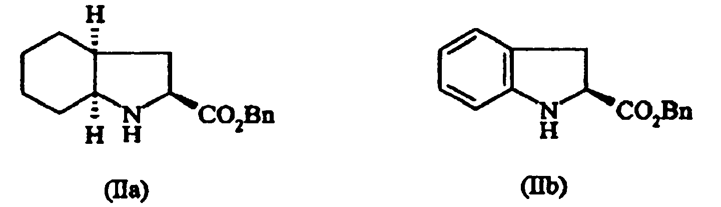 ou le sel d'addition de l'ester de formule (IIa) ou (IIb) avec un acide minéral ou organique,
avec le composé de formule (III) : 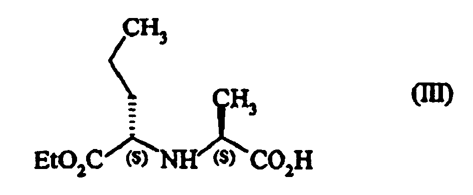 en présence d'un agent de couplage choisi parmi les réactifs et couples de réactifs suivants :
1-(3-diméthylaminopropyl)-3-éthyl-carbodiimide chlorhydrate / 1-hydroxybenzotriamle, et anhydride propanephosphonique,
en présence éventuelle de base,
pour conduire après hydrogénation catalytique en présence de palladium au perindopril de formule (I), que l'on transforme, si on le souhaite, en un sel pharmaceutiquement acceptable.

2. Procédé de synthèse selon la revendication 1 du perindopril sous sa forme de sel de tert-butylamine.

3. Procédé selon la revendication 1, **caractérisé en ce que** l'on part du composé de formule **(IIa).**

4. Procédé selon la revendication 1, **caractérisé en ce que** l'on part du composé de formule (IIb).

5. Procédé selon la revendication 3, **caractérisé en ce que** la réaction d'hydrogénation est effectuée sous une pression d'hydrogène inférieure à 10 bars.

6. Procédé selon la revendication 4, **caractérisé en ce que** la réaction d'hydrogénation est effectuée sous une pression d'hydrogène comprise entre 10 et 35 bars.

## Claims

1. Process for the industrial synthesis of perindopril of formula (I) 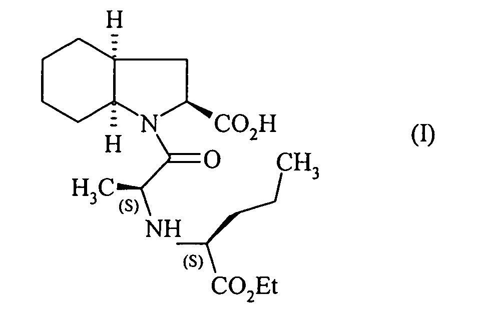 and pharmaceutically acceptable salts thereof, **characterised in that** the benzyl ester of formula (IIa) or (IIb): 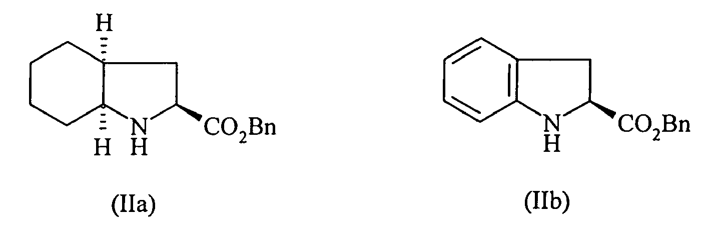 or an addition salt of the ester of formula (IIa) or (IIb) with a mineral acid or organic acid is reacted
with the compound of formula (III) : 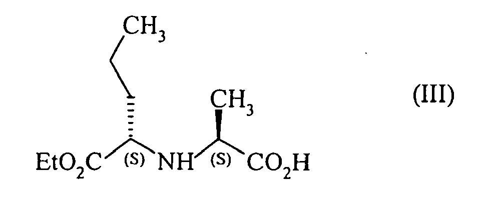 in the presence of a coupling agent selected from the following reagents and pairs of reagents :
1-(3-dimethylaminopropyl)-3-ethyl-carbodiimide hydrochloride / 1-hydroxybenzotriazole, and
propanephosphonic anhydride,
optionally in the presence of a base,
to yield, after catalytic hydrogenation in the presence of palladium, perindopril of formula (I), which is converted, if desired, into a pharmaceutically acceptable salt.

2. Process according to claim 1 for the synthesis of perindopril in the form of its tert-butylamine salt.

3. Process according to claim 1, **characterised in that** the compound of formula (IIa) is used as starting material.

4. Process according to claim 1, **characterised in that** the compound of formula (IIb) is used as starting material.

5. Process according to claim 3, **characterised in that** the hydrogenation reaction is carried out under a hydrogen pressure of less than 10 bars.

6. Process according to claim 4, **characterised in that** the hydrogenation reaction is carried out under a hydrogen pressure of from 10 to 35 bars.

## Patentansprüche

1. Verfahren zur technischen Synthese von Perindopril der Formel (I) 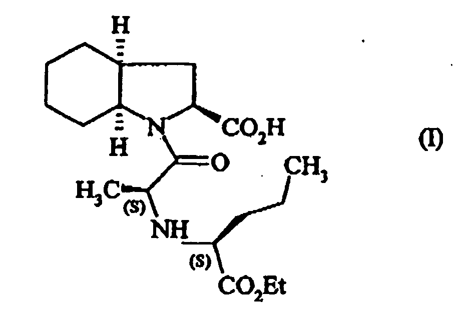 und von seinen pharmazeutisch annehmbaren Salzen, **dadurch gekennzeichnet, daß** man den Benzylester der Formel (IIa) oder (IIb): 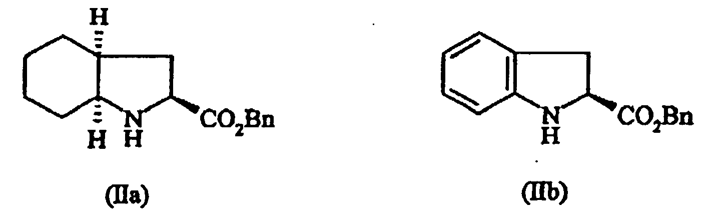 oder ein Additionssalz des Esters der Formel (IIa) oder (IIb) mit einer anorganischen oder organischen Säure
mit der Verbindung der Formel (III): 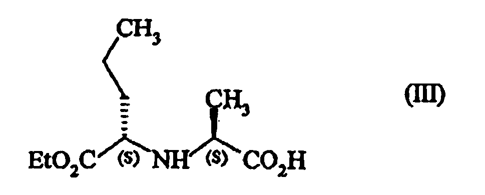 in Gegenwart eines Kupplungsmittels ausgewählt aus den folgenden Kupplungsreagenzien:
1-(3-Dimethylaminopropyl)-3-ethyl-carbodiimid-Hydrochlorid/1-Hydroxybenzotriazol und
Propanphosphonsäureanhydrid
gegebenenfalls in Gegenwart einer Base umsetzt,
so daß man nach der katalytischen Hydrierung in Gegenwart von Palladium Perindopril der Formel (I) erhält, welches man gewünschtenfalls in ein pharmazeutisch annehmbares Salz überführt.

2. Verfahren nach Anspruch 1 zur Synthese von Perindopril in Form seines tert.-Butylaminsalzes.

3. Verfahren nach Anspruch 1, **dadurch gekennzeichnet, daß** man von der Verbindung der Formel (IIa) ausgeht.

4. Verfahren nach Anspruch 1, **dadurch gekennzeichnet, daß** man von der Verbindung der Formel (IIb) ausgeht.

5. Verfahren nach Anspruch 3, **dadurch gekennzeichnet, daß** die Hydrierungsreaktion unter einem Wasserstoffdruck von weniger als 10 bar durchgeführt wird.

6. Verfahren nach Anspruch 4, **dadurch gekennzeichnet, daß** die Hydrierungsreaktion unter einem Wasserstoffdruck zwischen 10 und 35 bar durchgeführt wird.
